# EUROPEAN PATENT APPLICATION

(11) **EP 4 137 475 A1**
(43) Date of publication of application: **22.02.2023**
(21) Application number: 21192388.3
(22) Date of filing: 20.08.2021
(51) Int. Cl.: C07C 41/16, C07C 43/16, C07C 43/178

(54) **CHEMICAL PROCESS FOR VINYL ETHER SYNTHESIS**

(71) Applicant: Henkel AG & Co. KGaA, 40589 Düsseldorf (DE)
(72) Inventor: Brandt, Adrian, 45219 Essen (DE); Beck, Horst, 41470 Neuss (DE); Taden, Andreas, 40597 Düsseldorf (DE); Spiegelberg, Brian, 40627 Düsseldorf (DE); de Vries, Johannes Gerardus, 18055 Rostock (DE); Tin, Sergey, 18057 Rostock (DE)

(57) **Abstract**

The present invention relates to a method for producing at least one vinyl ether compound using at least one Iridium catalyst, at least one base and at least one solvent selected from ester and/or ether solvents. Such a vinyl ether compound can be polymerized and used in an adhesive, especially for the production of UV adhesives, cationic curing or 1-component or 2-component systems.

## Description

The present invention relates to a method for producing at least one vinyl ether compound using at least one Iridium catalyst, at least one base and at least one solvent selected from ester and/or ether solvents. Such a vinyl ether compound can be polymerized and used in an adhesive, especially for the production of UV adhesives, cationic curing or 1-component or 2-component systems.

UV adhesives and reactive adhesives are widely used in the art and are versatile adhesives used for many different applications, including but not limited to automotive industry, furniture, veneers, electronics and the like. Most of the existing processes for producing compounds suitable for this type of adhesive are not based on sustainable and environmentally friendly techniques.

Thus, there is need in the art, for alternative manufacturing processes that can overcome the known drawbacks and provide compounds in economically sufficient yield, especially towards a divinyl product avoiding any acetals, preferably based on renewable resources for sustainable adhesive systems with a good performance.

Therefore, the present invention is based on the inventors' finding that mono- and divinyl ether compounds, especially divinyl ether compounds are obtainable by a specific method starting from vinyl ester, preferably at least one bio-based substrate, in the presence of an Iridium catalyst, a base and a polar solvent. It was found that the ratio of produced mono- and divinyl ether compounds is in favor of the divinyl ether, as well as acetals as side products can be significantly reduced.

The present invention relates to a method for producing at least one vinyl ether compound, wherein the method comprises or consists of the steps:
reacting a vinyl ester with an alcohol having at least one hydroxy group, preferably at least two hydroxy groups, more preferably exactly two hydroxy groups in the presence of at least one Iridium catalyst, at least one base and at least one solvent selected from ester and/or ether solvents.

In a preferred embodiment, the present invention relates to a method for producing at least one vinyl ether compound of formula (I) 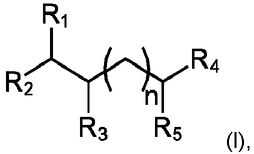 wherein
n is an integer of 0 to 5;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂;
wherein the method comprises or consists of the following steps:
   reacting a compound of formula (II) 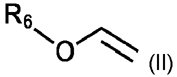
   wherein R₆ is -C(=O)R₁₂, and R₁₂ is an unsubstituted C₁-C₁₀ alkyl group; preferably the compound of formula (II) is vinyl acetate,
   with a compound of formula (III) 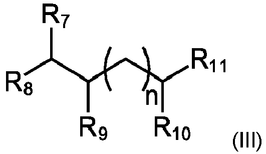 wherein
      n is an integer of 0 to 5;
      R₇, R₉ and R₁₁ are independently selected from -OH, -H or a substituted or unsubstituted C₁-C₁₀ alkyl or C₂-C₁₀ alkenyl group; and
      R₈ and R₁₀ are independently selected from -H or a substituted or unsubstituted C₁-C₁₀ alkyl or C₂-C₁₀ alkenyl group;
      with the proviso that at least two of R₇, R₉ and R₁₁ are -OH;
      in the presence of at least one Iridium catalyst, at least one base and at least one solvent selected from ester and/or ether solvents.

With this method a highly efficient synthesis towards bis-vinylethers from any kind of diol is presented. Diols with both primary and secondary OH-group like 1,3-butanediol, 1,4-pentanediol, 1,5-hexanediol can be converted successfully to the corresponding bis-vinyl ethers (yields higher than 90%) what is otherwise difficult to achieve due to the formation of cyclic acetals and linear acetals.

In various embodiments, in the compound of formula (I)
n is 1; and/or
R₁ is -OH or -O-CH=CH₂; and/or
R₂ is -CH3; and/or
R₃ is -H; and/or
R₄ is -OH or -O-CH=CH₂; and/or
R₅is -H;
wherein at least one of R₁ and R₄ is -O-CH=CH₂, preferably R₁ and R₄ are -O-CH=CH₂; and/or
wherein in formula (III)
n is 1; and/or
R₇ is -OH; and/or
R₈ is -CH3; and/or
R₉ is -H; and/or
R₁₀ is -H; and/or
R₁₁ is -OH.

In various embodiments, the reaction temperature of the method according to the invention is between 50 to 150 °C, preferably between 60 to 90°C or 90 to 120 °C.

In various embodiments, the reaction time of the method according to the invention is between 1 to 15 h, preferably between 4 to 10 h.

In various embodiments of the method according to the invention,
(i) the at least one Iridium catalyst is selected from Chlorobis(cyclooctene)iridium(I) dimer, Chloro-1,5-cyclooctadiene iridium(I) dimer,1,5-Cyclooctadiene(acetylacetonato)iridium(I), 1,5-Cyclooctadiene(hexafluoroacetylacetonato)iridium(I), 1,5-Cyclooctadiene(η5-indenyl)iridium(I), Di-µ-methoxobis(1,5-cyclooctadiene)diiridium(I), (Methylcyclopentadienyl)(1,5-cyclooctadiene)iridium(I), Chloro(1,5-cyclooctadiene)(1,10-phenanthroline)iridium(I) THF adduct, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate, more preferably cationic Ir(I) complexes like Chlorobis(cyclooctene)iridium(I) dimer, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate most preferably Chlorobis(cyclooctene)iridium(I) dimer and Bis(1,5-cyclooctadiene)iridium(I) tetrafluoroborate ; and/or
(ii) the at least one base is selected from sodium carbonate, potassium carbonate, cesium carbonate, potassium phosphate, sodium phosphate, sodium benzoate, potassium benzoate, sodium formate, potassium formate, sodium methoxide, potassium methoxide, sodium ethoxide, potassium ethoxide, sodium-tertbutoxide, potassium-tertbutoxide, sodium trimethylacetate, sodium acetate or potassium acetate, more preferably selected from sodium acetate or potassium acetate; and/or
(iii) the least one solvent is selected from lactons, like gamma-valerolacton, ether solvents, like cyclopentyl methyl ether, methyl tert-butyl ether, tetrahydrofuran, 2-methyl tetrahydrofuran, 3-methyl tetrahydrofuran, 2,5-dimethyltetrahydrofuran, 2,2,5,5-tetramethyltetrahydrofuran, most preferably the solvent is selected from tetrahydrofuran and 2-methyl tetrahydrofuran, especially the solvent is 2-methyl tetrahydrofuran.

In addition, the method according to the invention can be carried out under protective atmosphere (nitrogen, argon).

In various embodiments of the method according to the invention, the vinyl ester, especially the compound of formula (II), is added in molar excess over the alcohol, especially the compound of formula (III), preferably in a molar equivalent ratio of at least 1:1, (>1:1), more preferably of at least 2:1, most preferably in a ratio of 3:1 (vinyl ester: alcohol; especially formula (II):formula (III)).

In further embodiments of the method according to the invention, the vinyl ether being the reaction product of a vinyl ester and a alcohol having two hydroxy groups, especially compound of formula (I) is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

The details of one or more embodiments are set forth in the accompanying drawings and the description below. Other features, objects, and advantages will be apparent from the description, drawings and examples, and from the claims.

The following detailed description refers to, by way of illustration, specific details and embodiments in which the invention may be practiced. These embodiments are described in sufficient detail to enable those skilled in the art to practice the invention. Other embodiments may be utilized and structural and logical changes may be made without departing from the scope of the invention. The various embodiments are not necessarily mutually exclusive, as some embodiments can be combined with one or more other embodiments to form new embodiments.

Unless otherwise defined, all technical and scientific terms used herein have the same meaning as commonly understood by one of ordinary skill in the art. The singular terms "a", "an" and "the" include plural referents unless context clearly indicates otherwise.

"At least one", as used herein, means one or more, i.e. 1, 2, 3, 4, 5, 6, 7, 8, 9 or more of the referenced species. Similarly, "one or more", as used herein, relates to at least one and comprises 1, 2, 3, 4, 5, 6, 7, 8, 9 or more. In connection with a given species, the term does not relate to the total number of molecules, but rather to the type of species, e.g. vinyl ether compounds. In connected with amounts, the term relates to the total amount of the referenced species.

Numeric values specified without decimal places refer to the full value specified with one decimal place, i.e. for example, 99 % means 99.0 %, unless otherwise defined.

The terms "about" or "approximately" or "approx.", in connection with a numerical value, refer to a variance of ±10 %, preferably ±5 %, more preferably ±2 %, more preferably ±1 %, and most preferably less than ±1 %, with respect to the given numerical value.

When an amount, a concentration or other values or parameters is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

The present invention relates to a method for producing at least one vinyl ether compound, wherein the method comprises or consists of the steps:
reacting a vinyl ester with an alcohol having at least one hydroxy group, preferably at least two hydroxy groups, more preferably exactly two hydroxy groups in the presence of at least one Iridium catalyst, at least one base and at least one solvent selected from ester and/or ether solvents.

Preferably, the present invention relates to a method for producing at least one vinyl ether compound of formula (I) 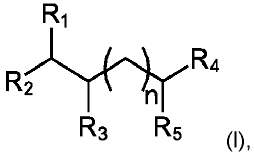 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂, preferably R₁ and/or R₄ is/are -O-CH=CH₂;
wherein the method comprises or consists of the following steps:
   reacting a compound of formula (II) 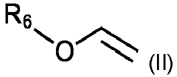
   wherein R₆ is -C(=O)R₁₂, or an unsubstituted C₁-C₁₀ alkyl or C₂-C₁₀ alkenyl group, preferably an unsubstituted C₁-C₆ alkyl group, most preferably an unsubstituted C₂ alkyl group; and R₁₂ is an unsubstituted C₁-C₁₀ alkyl group, preferably -CH₃,
   with a compound of formula (III) 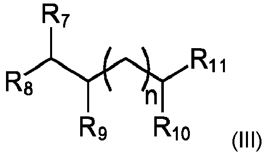 wherein
      n is an integer of 0 to 5, preferably 1, 2 or 3, most preferably 1;
      R₇, R₉ and R₁₁ are independently selected from -OH, -H or a substituted or unsubstituted C₁-C₁₀ alkyl or C₂-C₁₀ alkenyl group; and
      R₈ and R₁₀ are independently selected from -H or a substituted or unsubstituted C₁-C₁₀ alkyl or C₂-C₁₀ alkenyl group;
      with the proviso that at least two of R₇, R₉ and R₁₁ are -OH, preferably R₇ and R₁₁ are -OH;
      in the presence of at least one Iridium catalyst, at least one base and at least one solvent selected from ester and/or ether solvents.

In various embodiments of the method according to the invention, the alcohol, especially the compound of formula (III) is bio-based.

In general, unless otherwise stated, bio-based materials or biomaterials include or are preferably made of materials, chemicals and energy derived from renewable biological resources, for example from living organisms such as plants, bacteria or animals, without being limited to these. "Bio-based", as used in this context, thus refers to compounds/materials that can be obtained from biological, i.e. renewable, sources. One example for a compound of formula (III) is bio-based 1,4-pentane diol, typically obtained from levulinic acid, which is derived from the degradation of cellulose. An alternative route would be via furfural, furfuryl alcohol, levulinic acid to 1,4-pentane diole.

In one embodiment of the method according to the invention, vinyl ether(s) according to the invention are prepared from bio-based materials, preferably from bio-based diols, most preferably from bio-based 1,4-pentanediol. In various embodiments, compounds of formula (III) are bio-based diols, most preferably bio-based 1,4-pentanediol.

Typically, the term "C₁-C₁₀ alkyl" comprises linear and branched alkyl groups, wherein linear alkyl groups comprise 1 to 10, preferably 1 to 6 C-atoms and branched alkyl groups comprise 3 to 10, preferably 3 to 6 C-atoms. In addition, the term "C₂-C₁₀ alkenyl" comprises linear and branched alkenyl groups, wherein linear alkenyl groups comprise 2 to 10, preferably 2 to 6 C-atoms and branched alkenyl groups comprise 4 to 10, preferably 4 to 6 C-atoms.

In general, in substituted alkyl or alkenyl groups, each H can be independently substituted with a halogen group (e.g. -F, -CI, -Br or- I), -O-CH₃, -O-CH₂-CH₃, or -NO₂. It is however preferred that in various embodiments these groups are unsubstituted.

In various embodiments, in vinyl ether compounds of formula (I)
n is 1; and/or
R₁ is -OH or -O-CH=CH₂; and/or
R₂ is -CH3; and/or
R₃ is -H; and/or
R₄ is -OH or -O-CH=CH₂; and/or
R₅is -H;
wherein at least one of R₁ and R₄ is -O-CH=CH₂, preferably R₁ and R₄ are -O-CH=CH₂; and/or
wherein compound of formula (II) is vinyl acetate; and/or
wherein in formula (III)
n is 1; and/or
R₇ is -OH; and/or
R₈ is -CH3; and/or
R₉ is -H; and/or
R₁₀ is -H; and/or
R₁₁ is -OH.

In various embodiments of the method according to the invention, the compound of formula (II) is added in molar excess over the compound of formula (III), preferably in a molar equivalent ratio of at least 1:1, (>1:1), more preferably of at least 2:1, most preferably in a ratio of 3:1 (formula (II):formula (III)).

In various embodiments of the method according to the invention,
(i) the at least one Iridium catalyst is selected from Chlorobis(cyclooctene)iridium(I) dimer, Chloro-1,5-cyclooctadiene iridium(I) dimer,1,5-Cyclooctadiene(acetylacetonato)iridium(I), 1,5-Cyclooctadiene(hexafluoroacetylacetonato)iridium(I), 1,5-Cyclooctadiene(η5-indenyl)iridium(I), Di-µ-methoxobis(1,5-cyclooctadiene)diiridium(I), (Methylcyclopentadienyl)(1,5-cyclooctadiene)iridium(I), Chloro(1,5-cyclooctadiene)(1,10-phenanthroline)iridium(I) THF adduct, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate, more preferably cationic Ir(I) complexes like Chlorobis(cyclooctene)iridium(I) dimer, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate most preferably Chlorobis(cyclooctene)iridium(I) dimer and Bis(1,5-cyclooctadiene)iridium(I) tetrafluoroborate ; and/or
(ii) the at least one base is selected from sodium carbonate, potassium carbonate, cesium carbonate, potassium phosphate, sodium phosphate, sodium benzoate, potassium benzoate, sodium formate, potassium formate, sodium methoxide, potassium methoxide, sodium ethoxide, potassium ethoxide, sodium-tertbutoxide, potassium-tertbutoxide, sodium trimethylacetate, sodium acetate or potassium acetate, more preferably selected from sodium acetate or potassium acetate; and/or
(iii) the least one solvent is selected ester and/or ether solvents, preferably from cyclic ester and/or ether solvents, especially cyclic ether solvents, more preferably the solvent is selected from lactons, like gamma-valerolacton, ether solvents, like cyclopentyl methyl ether, methyl tert-butyl ether, tetrahydrofuran, 2-methyl tetrahydrofuran, 3-methyl tetrahydrofuran, 2,5-dimethyltetrahydrofuran, 2,2,5,5-tetramethyltetrahydrofuran, most preferably the solvent is selected from tetrahydrofuran and 2-methyl tetrahydrofuran, especially the solvent is 2-methyl tetrahydrofuran.

In various embodiments, the catalyst is added in amounts of 0.01 to 2 mol%, preferably in 0.1 to 1.5 mol%, more preferably in 0.5 to 1.1 mol% based on the amount of the alcohol, especially the alcohol of formula (III).

In various embodiments, the base is added in amounts of 1 to 100 mol%, preferably 5 to 70 mol%, more preferably 10 to 50 mol%, based on the amount of the alcohol of formula (III).

In various embodiments, the reaction temperature of the method according to the invention is between 50 to 150 °C, preferably between 60 to 90°C or 90 to 120 °C. In specific, non-limiting embodiments according to the invention, the reaction temperature is 80 °C or especially 100 °C.

In various embodiments, the reaction time of the method according to the invention is between 1 to 15 h, preferably between 4 to 10 h.

In various embodiments of the method according to the invention, the vinyl ether compound of formula (I) is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

In various embodiments of the method according to the invention, the vinyl ether being the reaction product of a vinyl ester and a alcohol having two hydroxy groups, especially vinyl ether compound of formula (I) is a mixture of divinyl ether and mono vinyl ether compounds, wherein the ratio of the primary mono vinyl ether compound(s) to the secondary mono vinyl ether compound(s) is at least 60 wt-%, preferably at least 70 wt-%, more preferably at least 80 wt-%, more preferably at least 90 wt-%, more preferably at least 95 wt-% based on the amount of the primary mono vinyl ether compound(s) and the secondary mono vinyl ether compound(s) in the mixture.

Generally, a higher reaction temperature leads to a higher yield of divinyl ether in comparison to mono vinyl ether. A lower temperature generally leads to a higher selectivity of the mixture of mono vinyl ether, i.e. of primary and secondary mono vinyl ether.

In various embodiments of the method according to the invention, the method comprises a further step of product purification. Examples include, without limitation filtration, column chromatography, vacuum evaporation/distillation, with evaporation/distillation being preferred. These steps are commonly known to the skilled person in the art.

In an embodiment of the methods according to the present invention, the compound of formula (I) is a vinyl ether compound of formula (IV) 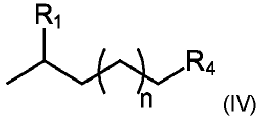 wherein
n is 0, 1, 2, 3, 4 or 5, preferably 1, 2 or 3, most preferably 1;
R₁ and R₄ are -OH or -O-CH=CH₂, with the proviso that at least one of R₁ and R₄ is -O-CH=CH₂, preferably R₁ and R₄ are -O-CH=CH₂; or
the vinyl ether compound is any one of the compounds of formulae (V) to (VII) 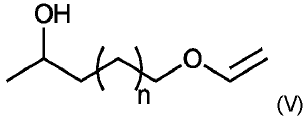 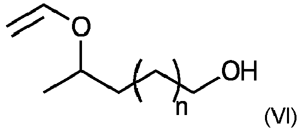 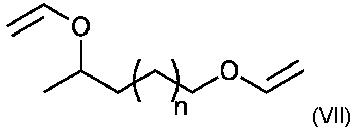 or a mixture thereof;
   wherein
   n is 0, 1, 2, 3, 4 or 5, preferably 1, 2 or 3, most preferably 1.

In various embodiments, the vinyl ether compound of formula (I) or (IV) according to the invention is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

In various embodiments, the vinyl ether compound of formula (I) or (IV) is a mixture of divinyl ether and mono vinyl ether compounds, wherein the ratio of the primary mono vinyl ether compound(s) to the secondary mono vinyl ether compound(s) is at least 60 wt-%, preferably at least 70 wt-%, more preferably at least 80 wt-%, more preferably at least 90 wt-%, more preferably at least 95 wt-% based on the amount of the primary mono vinyl ether compound(s) and the secondary mono vinyl ether compound(s) in the mixture.

The vinyl ether, especially the vinyl ether compound of formula (I) according to the invention can be used to obtained a polymer optionally together with at least one comonomer. Generally, all commonly known comonomers, which can undergo a reaction with a vinyl group are suitable. While cationic polymerization is preferred, thiol-ene reaction and polymerization is similarly possible. Radical polymerization is relevant for vinyl ether-based dispersions or when using vinyl ether monomers as comonomers in radical polymerization with (meth)acrylates

The at least one comonomer may be a further vinyl ether different from the produced vinyl ether, especially different from formula (I), e.g. an alkyl vinyl ether or a hydroxy(alkyl) vinyl ether, and/or vinyl lactam and/or vinyl amid and/or isocyanate terminated polyol and/or isocyanate terminated polyol endcapped with hydroxyalkyl vinyl ether, more preferably the at least one comonomer is selected from the group of methyl vinyl ether, ethyl vinyl ether, allyl vinyl ether, propyl vinyl ether, isopropyl vinyl ether, butyl vinyl ether, isobutyl vinyl ether, *tert*-butyl vinyl ether, 2-ethylhexyl vinyl ether (EHVE), hydroxyethyl vinyl ether (HEVE), hydroxybutyl vinyl ether or ethoxylated hydroxybutyl vinyl ether with 20-125 EO-units, aminopropyl vinyl ether, phenyl vinyl ether, benzyl vinyl ether, cyclohexyl vinyl ether, butandiol divinyl ether, diethyleneglycol divinyl ether, tri(ethylene glycol) methyl vinyl ether, triethylenglycol divinyl ether, dodecyl vinyl ether, octadecyl vinyl ether, cyclohexane dimethanol divinyl ether (CHDVE), cyclohexane dimethanol mono vinyl ether (CHMVE), trimethylolpropane trivinyl ether (TMPTVE), N-vinyl formamide, N-vinyl pyrrolidone, N-vinyl caprolactame (NVC) or liquid N-vinyl caprolactame (LNVC), N,N'-divinyl-2-imidazolidone, N-vinylimidazole, vinylcarbazol, or isocyanate terminated polyols or isocyanate terminated polyols that are further endcapped with hydroxyalkyl vinyl ether derived from toluene-2,4-diisocyanate (TDI), diphenylmethane diisocyanate (MDI), hexamethylene diisocyanate (HMDI), isophorone diisocyanate (IPDI), pentamethylene diisocyanate (PDI), dicyclohexylmethane-4,4'-diisocyanate (H12MDI), *meta*-tetramethylxylylene diisocyanate (TMXDI) or polymeric diphenylmethane diisocyanate (PMDI).

Here, more than one co-monomer can be present, preferably 2, 3, 4, 5, 6, 7, 8, 9, 10 or more, preferably selected from the list above, but not limited thereto.

Generally, all polymerization techniques, in which a vinyl group can undergo a polymerization reaction are suitable. For example, the mixture of monomers may be polymerized by free radical polymerization to form a vinyl ether polymer or copolymer, without being limited to these methods. Exemplary polymerization initiators include organic azo or peroxo compounds. Suitable polymerization initiators are widely known in the art and readily available. In various embodiments, the polymerization initiator comprises or consists of 2,2'-azobisisobutyronitrile (AIBN). Other suitable initiators include redox initiators, which are widely used and well-known in the art. In various preferred embodiments, the polymerization occurs by cationic polymerization or thiol-ene addition reaction and polymerization. Suitable cationic initiators are known to those skilled in the art and include, without limitation, Brønstedt acids, Lewis acids or super acid generators, such as iodonium or sulfonium salts, which can optionally be activated by UV irradiation.

The polymerization reaction can be carried out by using the vinyl ether, especially the vinyl ether compound(s) of formula (I) as defined above, and adding a cationic and/or radical initiator, as detailed above. Additional monomers that can optionally be added, such as further co-monomers, can be added during the polymerization process.

The polymerization can be carried out at elevated temperature, preferably at about 70°C, for several hours, preferably 1 to 12 hours.

After the polymerization is complete, the solvent can be removed by applying heat and/or vacuum to the mixture. Different heating temperatures and/or vacuum settings are suitable depending for example, on the used solvent. In various embodiments, the solvent is evaporated such that residual solvent content in the copolymer is between about 1 wt.-% to 35 wt.-%, preferably about 20 to 25 wt.-% relative to the reaction mixture.

After the polymerization is complete, remaining free monomers can be removed by applying heat and/or vacuum to the mixture. For this, suitable heating temperatures and/or vacuum settings can be applied depending on the type of monomer used, with such conditions being easily determinable for those skilled in the art.

The correspondingly obtained polymers can be used in adhesives, preferably UV adhesives. Such adhesive formulations can additionally contain one or more further components, which are commonly known to those skilled in the art. In specific embodiments, these additional components are selected from fillers, thickeners, silane additives, colorants, perfumes, preservatives, resins and mixtures thereof.

All embodiments and examples described above for the method according to the invention may also apply to other aspects according to the invention, and vice versa.

In the following, the invention is described in more detail by reference to the examples. It is understood that these examples are for illustrative purposes only and that it is not intended that the invention is limited thereto.

### EXAMPLES

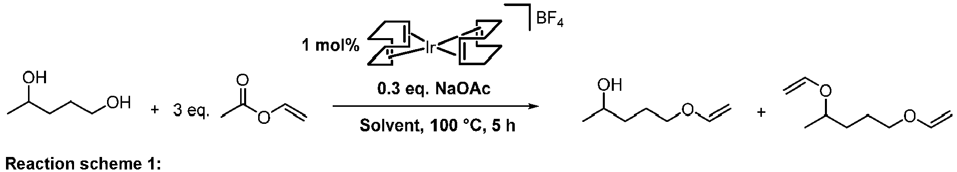

### Vinylation at 100 °C

A Schlenk tube (10 mL) is charged with a magnetic stirring bar, bis(1,5-cyclooctadiene)iridium(I)tetrafluoroborate (3·10⁻⁵ mol, 1 mol%, 15 mg) and anhydrous NaOAc (9·10⁻⁴ mol, 0.3 eq., 73.8 mg) under argon. Then 2.0 mL freshly distilled 2-MeTHF and 0.83 mL (0.009 mol, 3 eq.) degassed vinyl acetate are added. Afterwards, the degassed substrate 1,4-pentanediol (0.003 mol, 0.31 g) is added via syringe. The Schlenk tube is sealed under argon and transferred to a preheated oil bath at 100 °C in which the reaction mixture is stirred for 5 h. Subsequently, the mixture is cooling down to room temperature and then 2-MeTHF and the excess of vinyl acetate are removed under reduced pressure on the rotary evaporator. The product is purified by column chromatography using Et₂O/n-pentane (1:10/ v:v) as eluent on silica. After purification, the solvents are removed under reduced pressure on the rotary evaporator and the final compound is further dried at 40 °C at 140 mbar for 4 h.

The following table illustrates the conversion rate and results for various solvents:

| Solvent | Conversion | Yield monovinyl ether | Yield divinyl ether |
|---|---|---|---|
| Gamma-Valerolactone | 55% | 49% | 5% |
| 3-MeTHF | 95% | 55% | 18% |
| 2-MeTHF | 100% | 2% | 96% |
| THF | 99% | 21% | 62% |
| Cyclopentyl methyl ether | 100% | 3% | 45% |
| 2,5-Dimethylfuran | 100% | 31% | 49% |
| Methyl tert-butyl ether | 100% | 3% | 40% |
| Toluene | 100% | 4% | 20% |

With the non-polar solvent toluene the reaction resulted mainly an acetal formation instead of yielding the ethers. In contrast, the polar solvents reduced the side reaction of building the acetal and instead gained mainly the vinyl ethers. Especially the usage of tetrahydrofuran (THF) and 2-methyl tetrahydrofuran (2-MeTHF) lead to a high yield of divinyl ether, while almost avoiding any acetal as side product.

The following table illustrates the conversion rate, yield of divinylation and acetylation using various bases or no base for the above reaction with 2-MeTHF as solvent:

| Time [min] | | NaOAc | | | Na₂CO₃ | | | Without base | |
|---|---|---|---|---|---|---|---|---|---|
| | Conv. | Divinyl | Acetal | Conv. | Divinyl | Acetal | Conv. | Divinyl | Acetal |
| 60 | 70 | 9 | 0 | 84 | 7 | 2 | 57 | 0 | 45 |
| 120 | 75 | 15 | 1 | 85 | 13 | 3 | 80 | 0 | 68 |
| 180 | 100 | 34 | 1 | 94 | 20 | 3 | 100 | 0 | 90 |
| 240 | 100 | 65 | 2 | 100 | 32 | 3 | 100 | 0 | 91 |
| 300 | 100 | 96 | 2 | 100 | 37 | 3 | 100 | 0 | 91 |

While the reactions with a base lead to a divinylation and almost no acetal as side product, the same reaction without a base yields no divinyl product and the main reaction is towards the acetal.

## Claims

1. A method for producing at least one vinyl ether compound, wherein the method comprises or consists of the steps:
reacting a vinyl ester with an alcohol having at least one hydroxy group, preferably at least two hydroxy groups, in the presence of at least one Iridium catalyst, at least one base and at least one solvent selected from ester and/or ether solvents.

2. The method of claim 1, wherein the at least one vinyl ether compound is of formula (I) 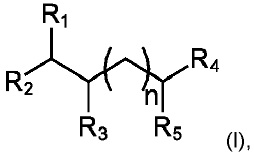 wherein
n is an integer of 0 to 5, preferably 1, 2 or 3, most preferably 1;
R₁, R₃ and R₄ are independently selected from -OH, -O-CH=CH₂, -H or a substituted or unsubstituted C₁-C₁₀ alkyl or C₂-C₁₀ alkenyl group; and
R₂ and R₅ are independently selected from -H or a substituted or unsubstituted C₁-C₁₀ alkyl or C₂-C₁₀ alkenyl group;
with the proviso that at least two of R₁, R₃ and R₄ are -OH or -O-CH=CH₂, and wherein at least one of R₁, R₃ and R₄ is -O-CH=CH₂, preferably R₁ and/or R₄ is/are -O-CH=CH₂;
and wherein the method comprises or consists of the following steps:
reacting a compound of formula (II) 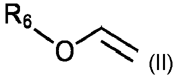
wherein R₆ is -C(=O)R₁₂; and R₁₂ is an unsubstituted C₁-C₁₀ alkyl group,
with a compound of formula (III) 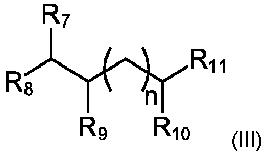 wherein
n is an integer of 0 to 5, preferably 1, 2 or 3, most preferably 1;
R₇, R₉ and R₁₁ are independently selected from -OH, -H or a substituted or unsubstituted C₁-C₁₀ alkyl or C₂-C₁₀ alkenyl group; and
R₈ and R₁₀ are independently selected from -H or a substituted or unsubstituted C₁-C₁₀ alkyl or
C₂-C₁₀ alkenyl group;
with the proviso that at least two of R₇, R₉ and R₁₁ are -OH, preferably R₇ and R₁₁ are -OH;
in the presence of at least one Iridium catalyst, at least one base and at least one solvent selected from ester and/or ether solvents.

3. The method of claim 2, wherein in formula (I)
n is 1; and/or
R₁ is -OH or -O-CH=CH₂; and/or
R₂ is -CH3; and/or
R₃ is -H; and/or
R₄ is -OH or -O-CH=CH₂; and/or
R₅ is -H;
wherein at least one of R₁ and R₄ is -O-CH=CH₂, preferably R₁ and R₄ are -O-CH=CH₂; and/or
wherein the compound of formula (II) is vinyl acetate; and/or
wherein in formula (III)
n is 1; and/or
R₇ is -OH; and/or
R₈ is -CH3; and/or
R₉ is -H; and/or
R₁₀ is -H; and/or
R₁₁ is -OH.

4. The method of any one of claims 1 to 3, wherein the reaction temperature is between 50 to 150 °C.

5. The method of any one of claims 1 to 4, wherein the at least one Iridium catalyst is selected from Chlorobis(cyclooctene)iridium(I) dimer, Bis(pyridine)(1,5-cyclooctadiene)iridium(I) hexafluorophosphate, Bis(1,5-cyclooctadiene)iridium(I) tetrakis[3,5-bis(trifluoromethyl)phenyl]borate, Bis(1,5-cyclooctadiene)iridium(I) tetrafluoroborate, Bis(acetonitrile)(1,5-cyclooctadiene)iridium(I) tetrafluoroborate, Bis(1,5-cyclooctadiene)iridium(I) hexafluorophosphate, (Tricyclohexylphosphine)(1,5-cyclooctadiene)(pyridine)iridium(I)hexafluorophosphate, most preferably Chlorobis(cyclooctene)iridium(I) dimer and Bis(1,5-cyclooctadiene)iridium(I) tetrafluoroborate.

6. The method of any one of claims 1 to 5, wherein the at least one base is selected from sodium acetate or potassium acetate.

7. The method of any one of claims 1 to 6, wherein vinyl ester, especially the compound of formula (II), is added in molar excess over the alcohol, especially compound of formula (III), preferably in a molar equivalent ratio of at least 1:1 or >1:1 (vinyl ester: alcohol).

8. The method of any one of claims 1 to 7, wherein the vinyl ether being the reaction product of a vinyl ester and a alcohol having two hydroxy groups, especially the vinyl ether of compound of formula (I), is a mixture of divinyl ether and mono vinyl ether compounds, preferably the mixture comprises at least 50 wt.-% divinyl ether, more preferably at least 60 wt.-%, more preferably at least 70 wt.-%, most preferably at least 80 wt.-%, based on the total weight of the mixture.

9. The method of any one of claims 1 to 8, wherein the solvent is selected from lactons, like gamma-valerolacton, cyclopentyl methyl ether, methyl tert-butyl ether, tetrahydrofuran, 2-methyl tetrahydrofuran, 3-methyl tetrahydrofuran, 2,5-dimethyltetrahydrofuran, 2,2,5,5-tetramethyltetrahydrofuran, more preferably the solvent is selected from tetrahydrofuran and 2-methyl tetrahydrofuran, especially the solvent is 2-methyl tetrahydrofuran.

10. The method of any one of claims 1 to 9, wherein the base is added in amounts of 1 to 100 mol%, preferably 5 to 70 mol%, more preferably 10 to 50 mol%, based on the amount of the alcohol of formula (III).

11. The method of any one of claims 1 to 10, wherein the alcohol is bio-based, preferably the compound of formula (III) is bio-based.
